# EUROPEAN PATENT SPECIFICATION

(11) **EP 1 123 046 B1**
(45) Date of publication and mention of the grant of the patent: **06.09.2006**
(21) Application number: 98949180.8
(22) Date of filing: 31.10.1998
(51) Int. Cl.: A61B 5/042

(54) **ENDOCARDIAL CATHETER SYSTEM FOR WAVELENGTH MEASUREMENT, MAPPING**
ENDOKARDIALES KATHETERSYSTEM ZUR WELLENLÄNGEMESSUNG, MEHRDIMENSIONALLEN DARSTELLUNG
SYSTEME DE CATHETER ENDOCARDIAQUE PERMETTANT DE MESURER UNE LONGUEUR D'ONDE ET D'EFFECTUER UN MAPPAGE

(43) Date of publication of application: 16.08.2001
(73) Proprietor: Abdolmohammadi, Akbar, 59160 Lomme (FR)
(72) Inventor: Abdolmohammadi, Akbar, 59160 Lomme (FR)
(74) Representative: Colens, Alain M.G.M.
(86) International application number: PCT/IB1998/001737
(87) International publication number: WO 2000/025685

(56) References cited:
- EP-A- 0 727 184
- EP-A- 0 832 602
- WO-A-95/00072
- WO-A-95/10236
- WO-A-97/25916

## Description

### 1) Background of the invention

The present invention relates generally to the field of the diagnosis and ablation using steerable vascular catheters .The invention is particularly directed to cardiac wavelength measurement and mapping.

### 2) Discussion of the related art:

Dr Cox & al ,have described an empirical surgical technique called the «MAZE operation »in which a series of incisions produce a segmentation of atrial tissue which could cure the majority of patients , however , the percentage of patients who do not benefit from this operation is far from negligible .

The efficiency of this operation is explained by the relation between the critical mass (the minimum width of a given atrial tissue under which the fibrillation is impossible) and the wavelength .The critical mass itself depends on the wavelength of the atrial tissue. In other words the greater the wavelength measured, the greater the width of tissue in which the fibrillation is impossible, this principal is represented by the following formula RWL = WL / CM ( where WL = wavelength , CM = critical mass, defined as the width of tissue below which the fibrillation is impossible , RWL= relative wavelength). As we can see there is a linear relation between the critical mass and the wavelength, the limit of the normal and pathological zone is defined by this line and the determinant element is the RWL .

This means that each time the width of the tissue is below the critical mass, the fibrillation stops and on the contrary , when it is above this value, it continues. This explains the failure of the MAZE operation in some patients.The MAZE operation is a serious surgical act with pain ,a long stay in hospital and multiple complications. Many have tried to use this technique by radiofrequency catheter ablation , however , the results have been disappointing . Not only does the procedure remain empirical (without measurement of the wavelength and the mass),but also, the creation of complete ablation lines is very difficult .

Undoubtedly , this is the result of a mediocre stability of ablation catheters actually available on the one hand, and the impossibility of effecting transmural lesions on the whole route with the same catheter and in the same conditions on the other .

It is obvious that the number of lines to be created by radio frequency ablation is dependent on the above parameters , so the measurement of the size of the atria (by echocardiography or by angiography), the wavelength and the appreciation of the anisotropy seems essential for an appropriate ablation of the fibrillation and some types of flutter.

The measurement of these parameters can also be helpful in the diagnosis of these arrhythmias when they arc undiagnosed by classical means .For example rare episodes and ,or,occurrence in some special circumstances . Also , for the evaluation of the mechanism of action of the drugs or other research programs this can be helpful .

The major problem for the measurement of the WL is the conduction velocity because the knowledge of the direction of the conduction velocity is essential for it's appropriate evaluation.

The WL has been measured in dog atria after thoracotomy by SMEETS & al using the parallel electrodes and adjusting these electrodes in a manner to have parallel recordings to confirm the direction of the propagation .

In clinical settings there is no known techniques for the measurement of the wavelength . The provision of a WL measuring and ablation catheter system that can successfully treat atrial fibrillation and some types of atrial flutters , by readily creating linear continuous lesions in the atria,would represent a definite advance in the treatment of these conditions .

Accordingly, it is a primary object of the invention to provide a multielectrode catheter shape,easily deployed directly ,or from main catheters or sheaths that provides shapes capable of adapting to varying contours of the atria and keeping a full stability until the end of the linear ablation procedure.Another object is to provide catheter shapes, which are easily deployed directly ,or from main catheters or sheaths that provide shapes capable of adapting to varying contours of the atria and manoeuvred to contact desired inner wall surfaces of the atria and sustain contact so that the WL measurement can be taken.

Document WO-A-95/00 072 discloses a mapping cardiac catheter comprising a head with a plurality of spaced apart inflatable arms, each arm carrying electrodes.

A further object is to provide a catheter system , which is easily deployed directly or from main catheters that provide shapes capable of adapting to varying contours of the heart chambers and sustaining contact so that the anisotropy of conduction can be appreciated .

Other objects and advantages of the invention will become apparent to those skilled in the art with the descriptions and figures of this specification .

### 3) Summary of the invention:

By means of the present invention, an array of distal working catheter shapes is provided ,which are easily deployed to contact the inner wall surfaces of the cardiac chambers in a manner that allows them to adapt completely to endocardial surfaces of the cardiac chambers and enables easy recording of impulses or the ablation procedures. The invention is defined in claim 1. In one aspect,
the invention features an atrial wavelength measuring system which includes :
- an elongated , flexible catheter shaft incorporating a plurality of electrical conductors , extending from the proximal extremity of the elongated , flexible catheter shaft to the distal extremity. There are a plurality of inflation lumens extending longitudinally through it, coupling screw syringes through proximal inflation ports to the distal inflation ports which are connected to inflatable structures of the stabilising system.
- a wavelength measuring head composed of: three arms having an expanded position and a contracted position .In the expanded position, the three arms are parallel and have a predetermined distance between them.

The expanded position is secured by thin cylindrical balloons of approximately one mm diameter made of a very strong, non-stretchable resinous material extended between the arms . Thereby providing a plurality of longitudinally spaced-apart electrodes on each arm and a plurality of conductors threaded wherein .
- a stabilising system which is a ring-like balloon made of a very compliant material bonded to the central arm In an expanded position the free part of the ring-like balloon backs up against the atrial wall and applies the WL measuring head to the opposite atrial wall under investigation.

In one embodiment the so-called arms are similar and constructed like classical catheters .

In another embodiment the wavelength measuring head includes :
a central rigid arm and a plurality of inflatable arms made of a very strong low non-stretchable material in which the precise interelectode distance is possible by accurate dosing of the inflation pressure.

In another embodiment the wavelength measuring head is composed of:
- one central classical catheter, whereon a plurality of longitudinally spaced-apart electrodes are provided.
- a plurality of inflatable arms on each side of the central arm having an extremely contracted position and an expanded position in which the lateral arms are perpendicular to the central arm . There are a plurality of electrodes on each arm having a predetermined distance between one another and the electrodes of the central arm in the expanded position .Each arm is connected to a screw syringe by an inflation lumen threaded in the elongated flexible member.
- a stabilising system which is a ring-form thin balloon made of a highly elastic material connected to a screw syringe by an inflation lumen.

### BRIEF DESCRIPTION OF THE DRAWINGS

FIG. 1 is a schematic illustration of a wavelength measuring system with a working head constructed of three longitudinally spaced-apart arms which are parallel in an expanded position.
FIG.2 is an enlarged cross-sectional view taken along the line 42-42 of the FIG 1.
FIG.3 is an enlarged view of the working head (part 31 of FIG 1) in a contracted position.
FIG. 4 is an enlarged lateral view of the head portion (31 of FIG 1) in a partly expanded position.
FIG. 5 is a schematic presentation of the position of the electrodes on the catheters of FIG 1
FIG. 6 is a pictorial representation of the catheter of FIG I in the left atrium with the stabilising system in an inflated position taking back up on the septum and pushing the ablation head to the wall under ablation.

### Detailed description of the invention :

The present invention relates to catheter probes for introduction into a chamber of the heart having blood therein and formed by a wall through a lumen leading to the chamber.

Generally, the catheter system is comprised of three main co-operating components including :
- a flexible elongated tubular member having proximal and distal extremities.
- a distal working catheter portion (called the working head ).
- a stabilising system which assures a high stability profile to the working head .

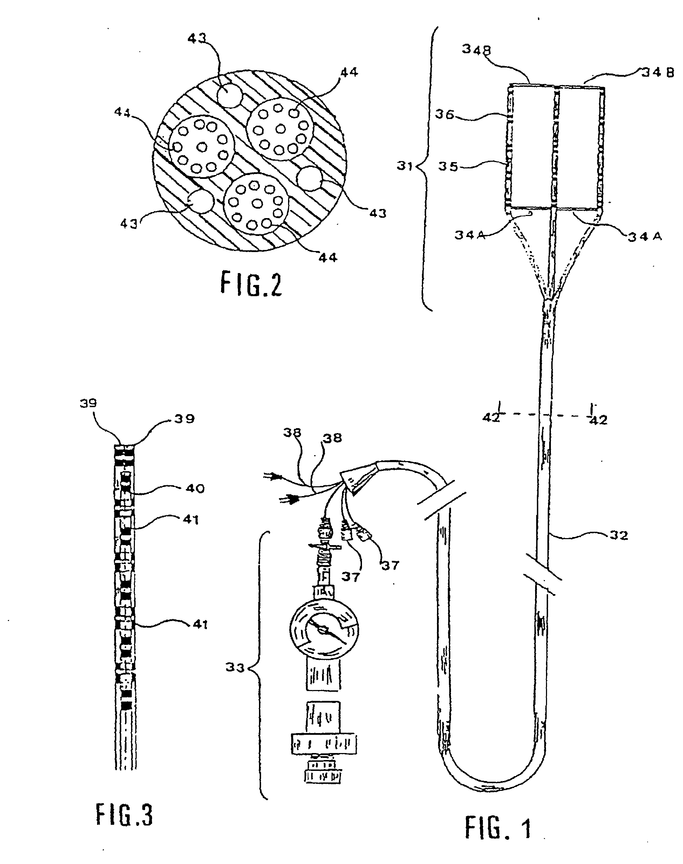

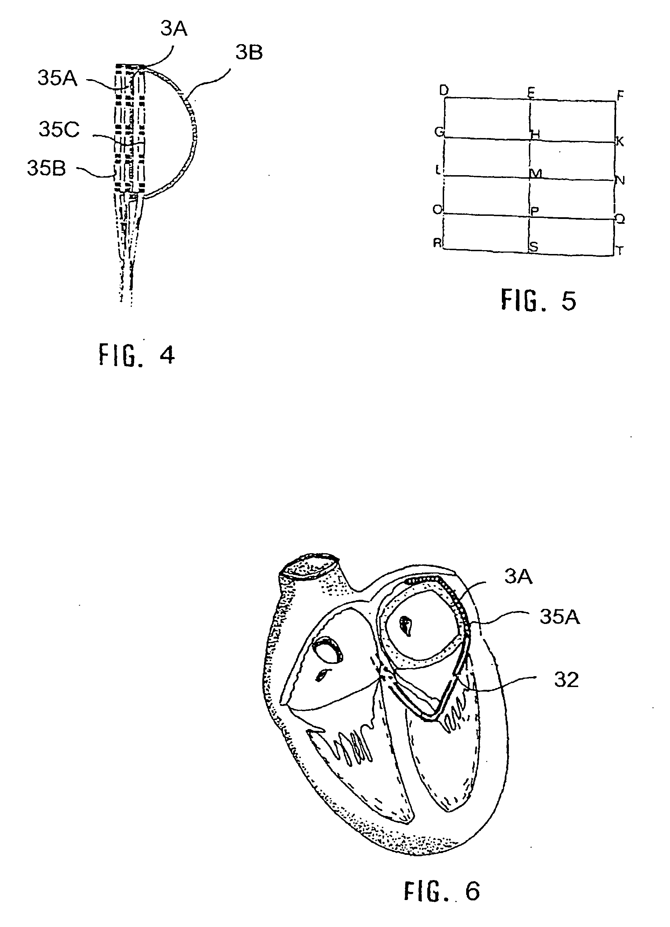

FIG. 1 is a general view of a wavelength measuring catheter comprising :
- an elongated, flexible, catheter shaft 32 with a plurality of inflation lumens 43 and a plurality of insulated conductors 44 extending from the proximal extremity to the distal extremity.
- a plurality of longitudinally extending spaced-apart arms having interconnected proximal extremities and carried by the distal extremity of the flexible elongated tubular member .The said arms are moveable between a contracted position (FIG 3) and an expanded position (FIG 1). The means are provided for moving the arms between a contracted and expanded position. In the expanded position, the wavelength measuring head 31 has a predetermined form allowing the wavelength measurement in a precise manner. A plurality of longitudinally spaced-apart electrodes 36 are provided on each arm and are connected to a plurality of conductors 44 extending through a lumen provided in the flexible elongated member .The conductors 44 are connected to cables 38 which are connected to a control console and power supply.
- a stabilising system is provided to apply the WL measuring head firmly to the heart wall under investigation . The said means is moveable between a contracted (small size) and an expanded position (FIG 4). When expanded the said stabilising means backs up on the contra lateral wall of the atrium and applies firmly the so called head to the wall under investigation.

In one embodiment (FIG 1) the so-called head 31 consist of::
- three arms , longitudinally spaced- apart , having interconnected proximal extremities in which the lateral arms are connected to the central one by cylindrical , thin , very low elasticity balloons in proximal 34 A and distal 34 B extremities. Said balloons are coupled to inflation ports 37 through inflation lumens extending along the length of catheter shaft 43. Balloons 34A and 34 B are inflatable with fluid preferably by a radiopaque solution which is injected by a syringe at the balloon inflation ports 37. By increasing the volume of the balloons the arms are separated and pressures are dosed in such a manner that the structure has three parallel arms with a distance of approximately I cm between each of the three arms (FIG 1).
- a ring-like , thin balloon 3b having a very high elasticity and attached to the central arm is coupled to an inflation port through an inflation lumen 43 extending along the length of catheter shaft and is inflatable with fluid preferably by a radiopaque solution which is injected by a syringe 33 at balloon inflation port 37. When the balloon is expanded it backs up on the opposite wall and applies the central arm firmly to the wall under investigation .

In this way , knowing the distances, the operator can stimulate the different electrodes and register the electrodes situated in the direction of the depolarisation
i.e. (see fig 5) let us suppose that pairs of electrodes are positioned at points :
D , E , F , G , H , K , L , M , N , O , P , Q , R , S , T with a distance :
   DE = EF = GH = HK = LM = MN = OP = PQ = RS = ST = 1 cm and
   DG = EH = FK = GL = HM = KN = LO = MP = NQ = OR = PS = QT = 0.5 cm

In this way , on stimulating at "D" we can measure the speed of conduction between E and F , between H and N, between M and T , between H and Q , between M and Q and by stimulating different points and combining the different measurements we can find the speed of conduction in different directions and on increasing the number of electrodes we can complete an extremely precise mapping of the conduction velocity.

## Claims

1. A measuring catheter system for introduction into an atrial chamber of a heart and the measurement of the conduction velocity and the anisotropy of the conduction comprising :
- an elongated, flexible, catheter shaft (32) having a proximal and a distal extremity with a plurality of inflation lumens (43) and insulated conductors (44) extending from the proximal extremity to the distal extremity,
- a flexible distal working catheter head (31) including a plurality of longitudinally extending spaced-apart arms (35) having interconnected proximal extremities and a plurality of electrodes (36) on each arm which are connected by a plurality of conductors (44) threaded in the arms and the elongated shaft (32) to a console,
the head (31) being capable of changing from a contracted position to an expanded position,
wherein in the expanded position the arms (35) are secured by a plurality of cylindrical balloons of approximately one mm diameter made of non-stretchable resinous material ,
the ballons extending between the arms and being connectable to a screw syringe (33) by said inflation lumens (43),
said balloons being inflatable with a fluid to assure a parallel position of the arms whereby the inter-electrode distances are predetermined allowing the measurement of the conduction velocity in several directions,
- a stabilising system comprising a ring-shaped balloon (3b) made of a compliant material and connectable to a screw syringe by one of the inflation lumen (43), said ring-shaped balloon being inflatable with fluid and allowing in the expanded position to insure a fixed position of the head (31) by pressing the head (31) to the atrial wall.

2. The system according to claim 1 wherein the fluid is a radiopaque solution.

3. The systems according to claim 2 further comprising ballon inflation ports (37) through which the radiopaque solution is injectable by a syringe (33).

## Patentansprüche

1. Ein Meßkathetersystem für die Einführung in eine atriale Kammer eines Herzens und für die Messung der Durchflußgeschwindigkeit und der Anisotrophie des Durchflusses, umfassend:
- einen länglichen, flexiblen Katheterschaft (32) mit einem proximalen und einem distalen Ende mit einer Anzahl von aufblasbaren Lumen (43) und isolierten Leitern (44), die sich vom proximalen Ende in Richtung auf das distale Ende erstrecken,
- einen flexiblen distalen Arbeitskatheter-Kopf (31) mit einer Anzahl von sich in Längsrichtung erstreckenden, voneinander beabstandeten Armen (35), deren proximale Enden miteinander verbunden sind, und mit einer Anzahl von Elektroden (36) an jedem Arm, die durch eine Anzahl von Leitern (44), die in die Arme und in den länglichen Schaft (32) in eine Konsole eingeschraubt sind, miteinander verbunden sind,
wobei der Kopf (31) von einer kontrahierten Position in eine expandierte Position wechseln kann,
wobei in der expandierten Position die Arme (35) durch eine Anzahl zylinderförmiger Ballons von ungefähr einem Millimeter Durchmesser gesichert werden, die aus einem nicht dehnbaren harzhaltigen Material bestehen,
wobei sich die Ballons zwischen den Armen erstrecken und über die besagten aufblasbaren Lumen (43) mit einer Schraub-Spritze (33) verbindbar sind,
wobei die besagten Ballons mit einer Flüssigkeit expandierbar sind, um eine parallele Position der Arme zu sichern, wodurch die Abstände zwischen den Elektroden vorgegeben sind und **dadurch** die Messung der Durchflußgeschwindigkeit in mehreren Richtungen erlauben,
- ein Stabilisierunssystem mit einem ringförmigen Ballon (3b), der aus einem nachgiebigen Material besteht und über einen der aufblasbaren Lumen (43) mit einer Schraub-Spritze verbindbar ist, wobei der besagte ringförmige Ballon durch eine Flüssigkeit expandierbar ist und in der expandierten Position die Sicherstellung einer örtlich festgelegten Position des Kopfes (31) **dadurch** ermöglicht, daß er den Kopf (31) gegen atriale Wand preßt.

2. Das System gemäß Ansprunch 1, wobei die Flüssigkeit eine radio-opaque Lösung ist.

3. Die Systeme gemäß Anspruch 2, die weiterhin ballon-dillatierbare Ports (37) umfassen, über die die radio-opaque Lösung durch eine Spritze (33) injizierbar ist.

## Revendications

1. Système de cathéter pour introduction dans une cavité atriale du coeur et la mesure de la vitesse de conduction et de l'anisotropie de conduction comprenant:
- un corps de cathéter, flexible, allonge (32) ayant des extrémités proximales et distales avec plusieurs lumières de gonflage (43) et des conducteurs isolés (44) sétendant de l'extrémité proximale à l'extrémité distale.
- une tête de mesure distale (31) comprenant plusieurs bras s'étendant longitudinalement (35) et ayant des extrémités interconnectées et sur chaque bras plusieurs électrodes (36) qui sont connectées par plusieurs conducteurs (44) incorporés dans les bras et le corps du cathéter (32) à une console,
la tête de mesure (31) étant capable de changer de forme et cela d'une forme contractée jusqu 'à une forme étendue, dans cette forme étendue les bras (35) étant soutenus par plusieurs ballons cylindriques d'approximativement 1 mm de diamètre fabriqué d'un matériau résineux non-élastique, ces ballons étant étendus entre les bras et étant connectés à une seringue à vis (33) par lesdites lumières de gonflage (43),
lesdits ballons étant gonflés par un fluide pour assurer une position parallèle aux bras dans laquelle les distances inter-electrodes sont prédéterminées permettant ainsi la mesure de la vitesse de conduction dans plusieurs directions,
- un système de stabilisation comprenant un ballon en forme de cercle (3b) fabriqué d'un matériau souple et connectable à une seringue à vis, par une des lumières de gonflage (43) ce ballon circulaire étant gonflable avec du fluide permettant d'avoir une position étendue qui assure une stabilité parfaite à la tête (31) en poussant celle-ci à la paroi atriale.

2. Le système selon la revendication précédente dans lequel le fluide est une solution radio-opaque.

3. Le système selon la revendication 2 comprenant en outre des orifices de gonflage du ballon (37) à travers lesquels la solution radio-opaque est injectée par une seringue (33).
